# EUROPEAN PATENT SPECIFICATION

(11) **EP 3 873 889 B1**
(45) Date of publication and mention of the grant of the patent: **11.02.2026**
(21) Application number: 18938668.3
(22) Date of filing: 29.10.2018
(51) Int. Cl.: C07D 319/12, C08G 63/06, C08G 63/78, C08G 63/91, C08J 11/12, C08J 11/24, C08J 11/26

(54) **GLYCOLIDE PRODUCTION FROM METHYL POLYGLYCOLATE**
HERSTELLUNG VON GLYKOLID AUS METHYLPOLYGLYKOLAT
PRODUCTION DE GLYCOLIDE À PARTIR DE POLYGLYCOLATE DE MÉTHYLE

(43) Date of publication of application: 08.09.2021
(73) Proprietor: Pujing Chemical Industry Co., Ltd, Shanghai 201102 (CN)
(72) Inventor: LIU, Wei, Shanghai 201102 (CN)
(74) Representative: V.O.
(86) International application number: PCT/CN2018/112468
(87) International publication number: WO 2020/087217

(56) References cited:
- EP-A1- 1 553 076
- WO-A1-99/19378
- WO-A2-02/070508
- CN-A- 105 272 958
- CN-A- 107 868 076
- JP-A- 2000 119 269
- US-A1- 2012 289 713

## Description

The present invention relates to glycolide production from a methyl polyglycolate or methyl polyglycolate product.

### BACKGROUND OF THE INVENTION

Methyl polyglycolate has good biocompatibility, excellent gas barrier properties, excellent mechanical properties, and excellent biodegradability. It is a polymer material with great potential and green environmental protection, which is in line with the development of today's society. The direction of demand is towards the wide use of methyl polyglycolate in biomedical devices, shale gas mining, packaging materials, and other fields.

At present, all commercially available high molecular weight methyl polyglycolate products are prepared by ring-opening polymerization of glycolide. Due to thermal degradation and biodegradability of methyl polyglycolate, larger quantities of methyl polyglycolate waste products have been produced from, for example, scraps when materials are trimmed or molded, and disposable packaging materials and supplies that have been used or degraded in various ways are produced in manufacturing, processing, and uses of methyl polyglycolate. Although methyl polyglycolate can be eventually naturally degraded into water and carbon dioxide, improper handling will not only cause temporary environmental problems, but also waste resources, when these wastes are too concentrated for natural degradation. Low resource utilization and the loss of the polymer during the degradation process contribute to the current high price of methyl polyglycolate product.

Although many patents have reported the preparation of methyl polyglycolate by glycolide, there have been few reports on the recycling of methyl polyglycolate product. This is due mainly to the varying qualities, and varying degradation degrees of methyl polyglycolate which is hard to recover and recycle. Therefore, there is still a need for a stable, repeatable, effective, and low-cost industrial process for recycling various recovered methyl polyglycolate and methyl polyglycolate product of different qualities. Chinese patent CN101851227B discloses the preparation of glycolide by catalytic pyrolysis of polyglycolic acid as a raw material under vacuum at 150-250 °C, and the crude product of the obtained glycolide has a low purity so that it needs to undergo three recrystallizations, resulting in a glycolide yield as low as 53-60 wt%, such that the utilization rate of the raw material is very low. Therefore, the foregoing method does not achieve efficient recovery of polyhydroxyethanol raw materials to prepare high-purity glycolide.

Chinese patent application CN103781833A reports a method for recovering glycolide from polyglycolide. Specifically, the polymer in a molten form is first combined with a hydrolysis medium, and begins to be hydrolyzed to a polyglycolic acid oligomer, and then the oligomer is cyclized and depolymerized to form glycolide. The method is suitable for polyglycolide having a number average molecular weight of 134 to 10,000 g/mol, and is not suitable for high molecular weight polyglycolide. In addition, the residual hydrolysis medium has a great influence on the quality and purity of the glycolide product.

US 2012/289713 A1 discloses a method for producing glycolide, comprising the steps of: heating a mixture containing glycolic acid oligomer, a high-boiling point polar organic solvent having a boiling point of 230 to 450 °C, and a tin compound under normal or reduced pressure until the temperature at which the glycolic acid oligomer is depolymerized, to thereby dissolve the glycolic acid oligomer in said solvent; heating a solution, in which the glycolic acid oligomer is dissolved, under normal or reduced pressure until the temperature at which the glycolic acid oligomer is depolymerized, to thereby form glycolide by depolymerization of the glycolic acid oligomer in the solution; and co-distilling off the high-boiling point polar organic solvent and the formed glycolide.

EP 1553076 A1 describes a process to produce glycolide wherein, in a first step, methyl glycolate is polymerized to polyglycolic acid (having a weight average molecular weight of 60,000) using stannic chloride as catalyst. The so-produced polyglycolic acid was then depolymerized by heating to 250 °C in triethylene glycol dimethyl ether to, thereby, from glycolide.

The above methods cannot meet the need for high-efficiency utilization of methyl polyglycolate and its products from a wide variety of sources, of different qualities and different degrees of degradation to obtain high-purity glycolide products. Therefore, efficient and convenient recovery of high-quality glycolide from methyl polyglycolate and methyl polyglycolate product is a problem that prior researchers have not overcome.

### SUMMARY OF THE INVENTION

The present invention relates to a process for producing glycolide from methyl polyglycolate or a methyl polyglycolate product, as well as to a process for producing glycolic acid oligomer from methyl polyglycolate or a methyl polyglycolate product.

In accordance with the present invention, a process of preparing a glycolide product from a methyl polyglycolate or a methyl polyglycolate product is provided, wherein the process comprises:
(a) depolymerizing a methyl polyglycolate or methyl polyglycolate product in the presence of a depolymerization agent, whereby a depolymerized product is generated, wherein the depolymerized product comprises glycolate, glycolate polymer or glycolate prepolymer;
(b) repolymerizing the depolymerized product, whereby a repolymerized mixture is generated, wherein the repolymerized mixture comprises a glycolic acid oligomer; and
(c) pyrolyzing the repolymerized mixture, whereby a glycolide product is prepared.

The methyl polyglycolate or the methyl polyglycolate product may have a weight average molecular weight of 1,000-1,000,000.

Unless specified otherwise, the weight average molecular weight of those materials employed in the processes of the present invention, and that of the products generated throughout the processes, is determined by gel permeation chromatography (GPC) as described herein below.

The depolymerization agent may comprise one or more hydroxyl groups and comprises an alkyl alcohol, an alkyl alkyd or an alkyl alkanoate. The alkyl alcohol may comprise one hydroxyl group, preferably methanol. The alkyl alkyd may comprise one or more hydroxyl groups and one or more carboxyl groups at a number ratio of the hydroxyl groups to the carboxyl groups in the range from 0.1:1 to 10:1, preferably 1:1, more preferably glycolic acid. The alkyl alkanoate may comprise one or more hydroxyl groups and one or more ester groups at a number ratio of the hydroxyl groups to the ester groups in the range from 0.1:1 to 10:1, preferably a ratio of 1:1, more preferably methyl glycolate.

The depolymerization step (a) may be carried out at 25-260 °C, preferably 30-190 °C, more preferably 40-120 °C, for 0.5-48 hours, preferably 1-36 hours, more preferably 2-24 hours.

The process may further comprise applying microwave irradiation to control temperature in the depolymerization step (a) so that depolymerization is accelerated.

The depolymerization step (a) may be carried out in the presence of a rare earth metal catalyst. The rare earth metal catalyst may be a metal oxide, a rare earth metal inorganic salt or a rare earth metal complex comprising lanthanum (La), cerium (Ce), praseodymium (Pr), neodynium (Nd), promethium (Pm), strontium (Sr), samarium (Sm), europium (Eu), gadolinium (Gd), terbium (Tb), dysprosium (Dy), holmium (Ho), erbium (Er), thulium (Tm), ytterbium (Yb), lutetium (Lu), yttrium (Y), scandium (Sc), or a combination thereof. The rare earth metal catalyst may be present in an amount of 0.0001-5 wt%, 0.0006-4 wt%, more preferably 0.001-2 wt%, based on the weight of the methyl polyglycolate or the methyl polyglycolate product.

The repolymerization step (b) may be carried out in the presence of a repolymerization catalyst. The repolymerization catalyst may be selected from the group consisting of zinc-based compounds, tin-based compounds, lanthanide-based compounds, and titanium-based compounds.

The repolymerization step (b) may be carried out at 160-250 °C and under an absolute pressure of 0.05-50.00 kPa.

The glycolic acid oligomer may have a weight average molecular weight of 4,000-80,000 and a free acid content of less than 2 wt%, the free acid content being measured as described herein below.

The pyrolysis step (c) may be carried out at 230-280 °C and an absolute pressure of 0.05-30.00 kPa.

The pyrolysis step (c) may be carried out in the presence of a viscosity reducer. The viscosity reducer may be present in an amount of 5-500 wt%, preferably 10-100 wt%, more preferably 15-60 wt%, based on the weight of the methyl polyglycolate or the methyl polyglycolate product. The viscosity reducer may have a boiling point greater than 330 °C. The process may further comprise distilling the glycolide product without the viscosity reducer.

The viscosity reducer may be a polyether polyol, preferably a polyethylene glycol, polypropylene glycol or polybutylene glycol, more preferably a polyethylene glycol having a weight average molecular weight between 1,500 and 20,000, most preferably a polyethylene glycol having a weight average molecular weight of between 1,500 and 8,000.

The viscosity reducer may be a hydrocarbon mixture, preferably a mixture having a weight average molecular weight of less than 25,000, more preferably a hydrocarbon mixture having a weight average molecular weight of 1,000-15,000, most preferably 1,500-8,000, and may be present in an amount of 5-500 wt%, preferably 10-100 wt%, more preferably 15-60 wt%, based on the weight of the methyl polyglycolate or the methyl polyglycolate product.

The pyrolysis step (c) may have a pyrolysis conversion ratio of more than 90% of the glycolic acid oligomer and generate an incompletely converted glycolide acid oligomer.

The process may further comprise discharging the incompletely converted glycolic acid oligomer as a residue at 230 °C with a dynamic viscosity less than 1 Pa·s, wherein the dynamic viscosity is measured using a rotational viscometer at 230°C.

The glycolide product prepared by the above-described process of the present invention may have a free acid content of less than 2%.

In accordance with a further aspect of the present invention, a process for preparing a glycolic acid oligomer from a methyl polyglycolate or a methyl polyglycolate product is provided, wherein the process comprises:
(a) depolymerizing a methyl polyglycolate or methyl polyglycolate product in the presence of a depolymerization agent, whereby a depolymerized product is generated, wherein the depolymerized product comprises glycolate, glycolate polymer or glycolate prepolymer; and
(b) repolymerizing the depolymerized product, whereby a glycolic acid oligomer is prepared.

The glycolic acid oligomer may have a weight average molecular weight of 4,000-80,000. The glycolic acid oligomer may have a free acid content of less than 2 wt%.

### DETAILED DESCRIPTION OF THE INVENTION

The present invention relates to a process of producing a glycolide product from methyl polyglycolate or a product made from methyl polyglycolate. The inventors have surprisingly discovered that a glycolic acid oligomer having a weight average molecular weight of 4,000-80,000 may be obtained by depolymerizing and repolymerizing methyl polyglycolate and may be used to produce high-purity glycolide by pyrolysis. After depolymerization, repolymerization and pyrolysis, the glycolide products are produced steadily and solve the above-discussed problems of resource waste and waste disposal in the production, manufacture and use of methyl polyglycolate.

Recovered methyl polyglycolate (including, but not limited to, waste in the production of methyl polyglycolate, trim in the processing of methyl polyglycolate, and polyglycolate products that have begun to degrade after use) have a wide molecular weight range. The weight average molecular weight range is approximately 1,000-1,000,000. The direct pyrolysis of these raw materials of different qualities is difficult to do while also ensuring a constant product quality. Methyl polyglycolates having very large molecular weight and very high melt viscosity are therefore difficult to process in the melt. It is difficult to reach a pyrolysis conversion rate greater than 90 wt% with very high molecular weight methyl polyglycolates. After pyrolysis, the remaining unconverted raw materials are discharged as a residue. When the molecular weight of the starting material for the pyrolysis is very high, more residues are produced due to slagging and, as mentioned above, the efficiency of the pyrolysis is very low due to the high viscosity.

The terms "depolymerized product" comprises glycolate, a glycolate polymer, a glycolate prepolymer, or a combination thereof.

The terms "glycolic acid oligomer" and "glycolate prepolymer" are used herein interchangeably and refer to a polymer comprising glycolic acid or glyoclate repeating units and having a weight average molecular of 4,000-8,000.

Unless stated otherwise, all molecular weight values are g/mol, and all average molecular weight values are number-average molecular weight values.

According to the process of the present invention, methyl polyglycolate or a methyl polyglycolate product is first depolymerized to a depolymerized product. The depolymerized product may be a glycolate, a glycolate polymer, a glycolate prepolymer, or a combination thereof. The depolymerized product may have a weight average molecular weight of less than 4,000. Depending on the extent of the depolymerization reaction, a glycolic acid monomer may be obtained if further depolymerization is accomplished.

The depolymerization may be carried out in the presence of a depolymerization agent. The depolymerization agent may comprise one or more hydroxyl groups. The depolymerization agent may comprise an alkyl alcohol, an alkyl alkyd or an alkyl alkanoate.

The alkyl alcohol contains one or more hydroxyl groups. The alkyl alcohol is preferably an alkyl alcohol having only one hydroxyl group. For example, the alkyl alcohol may be methanol.

The alkyl alkyd may comprise one or more hydroxyl groups and one or more carboxyl groups. In the alkyl alkyd, the number ratio of the hydroxyl groups to the carboxyl groups in the alkyl alkyd may be from 0.1:1 to 10:1. The number ratio is preferably 1:1. For example, the alkyl alkyd is glycolic acid.

The alkyl alkanoate may comprise one or more hydroxyl groups and one or more ester groups. In alkyl alkanoate, the number ratio of the hydroxyl group to the ester groups may be from 0.1:1 to 10:1. The number ration is preferably 1:1. For example, the alkyl alkanoate is methyl glycolate.

The alkyl alcohol, alkyl alkanoate, and alkyl alkyd added to the depolymerization reaction as depolymerization agents may remain in subsequent reactants and participate in the pyrolysis reaction to produce glycolide. These depolymerization agents do not affect the purity of the final glycolide product. At the same time, the aforesaid preferred methanol, glycolic acid, and methyl glycolate are preferentially distilled out during the high temperature pyrolysis to generate glycolide due to their lower boiling point, and do not remain in the glycolide. Therefore, the purity of the glycolide product is high. If the recovered methyl polyglycolate or methyl polyglycolate product is contaminated with sediment, the sediment can be simply washed and sieved with water.

Rare earth metal catalysts may be added to accelerate the depolymerization rate of high molecular weight methyl polyglycolate at lower temperatures during the depolymerization. The rare earth metal catalyst may be a metal oxide, a rare earth metal inorganic salt or a rare earth metal complex, each comprising lanthanum (La), cerium (Ce), praseodymium (Pr), neodynium (Nd), promethium (Pm), strontium (Sr) samarium (Sm), europium (Eu), gadolinium (Gd), terbium (Tb), dysprosium (Dy), holmium (Ho), erbium (Er), thulium (Tm), ytterbium (Yb), lutetium (Lu), yttrium (Y), scandium (Sc), or a combination thereof. The rare earth metal catalyst may comprise ruthenium, cobalt, rhodium, nickel, or a combination thereof. The rare earth metal catalyst may be an oxide of ruthenium, cobalt, rhodium, nickel, or a combination thereof. The rare earth metal catalyst may be present in an amount of 0.0001-5 wt%, 0.0006-4 wt%, more preferably 0.001-2 wt%, based on the weight of the methyl polyglycolate or the methyl polyglycolate product.

The depolymerization reaction temperature may be 25-260 °C, preferably 30-190 °C, more preferably 40-120 °C. The depolymerization reaction time may be 0.5-48 hours, preferably 1-36 hours, more preferably 2-24 hours. An increase in the reaction temperature promotes the depolymerization reaction.

The depolymerization may be carried out with microwave irradiation. At a relatively low heating temperature, intense microwave irradiation facilitates faster depolymerization, especially for methyl polyglycolate product having a high molecular weight. A temperature-controlled microwave equipment may be used to emit microwave irradiation for depolymerization. The emitted microwave output power may be about 1200-1600 W, for example, about 1400 W. The microwave is applied throughout the entire depolymerization process.

The depolymerized product is repolymerized to form a repolymerization mixture. The repolymerization mixture comprises a glycolic acid oligomer. The repolymerization reaction may be carried out at 100-300 °C, 150-300 °C or 160-250 °C and under an absolute pressure of 0.01-60.00 kPa or 0.05-50.00 kPa. The glycolic acid oligomer may have a weight average molecular weight of 4,000-80,000, and/or a free acid content of less than 2 wt%.

A glycolic acid oligomer having a weight average molecular weight in the range of 4,000 to 80,000 is desirable for producing glycolide with high purity and yield. The impurities and the depolymerization agent may be removed from the glycolic acid oligomer by distillation.

A repolymerization catalyst may be used to promote repolymerization. The repolymerization catalyst may be selected from the group consisting of zinc-based compounds, tin-based compounds, lanthanide-based compounds, and titanium-based compounds. A zinc-based, tin-based, antimony-based or titanium-based compound may be a compound comprising zinc, tin, antimony or titanium in any form, for example, a salt. Because the catalyst used for preparing the methyl polyglycolate and methyl polyglycolate product is not finally separated, the addition of a conventional polymerization catalyst such as a zinc-based, tin-based, antimony-based or titanium-based compound is no longer important in the repolymerization step.

A glycolic acid oligomer may be prepared by depolymerization and repolymerization. A glycolic acid oligomer having a lower free acid content can be obtained, thereby further reducing the free acid content to be 2% or less in the glycolide product prepared after pyrolysis. In addition, in the repolymerization reaction, impurities such as a depolymerization agent are separated by distillation so that high-purity glycolide can be obtained. The repolymerization process is not only advantageous for obtaining a glycolic acid oligomer having a weight average molecular weight of 4,000 to 80,000, but also advantageous for further purifying the intermediate product, thereby finally increasing the yield and purity of the glycolide.

In the pyrolysis reaction, repolymerization mixture is pyrolyzed and a glycolide product is obtained. The pyrolysis may be carried out at a temperature of 200-300 °C or 230-280 °C under an absolute pressure of 0.01-50 kPa or 0.05-30 kPa for 0.1-5 hours or 0.5-2 hours. No solvent may be added in the pyrolysis reaction.

A viscosity reducer may be added to facilitate the pyrolysis reaction. The viscosity reducer may have a boiling point greater than about 300, 330, 350 or 400 °C to enhance the melt flow. The viscosity reducer may be present in an amount of 5-500 wt%, preferably 10-100 wt%, more preferably 15-60 wt%, based on the weight of the methyl polyglycolate or the methyl polyglycolate product. The viscosity reducer may be a polyether polyol, preferably a polyethylene glycol, polypropylene glycol or polybutylene glycol, more preferably a polyethylene glycol having a weight average molecular weight of 1,500-20,000, most preferably a polyethylene glycol having a weight average molecular weight of 1,500-8,000. The viscosity reducer may be a hydrocarbon mixture, preferably a mixture having a weight average molecular weight of less than 25,000, more preferably a hydrocarbon mixture having a weight average molecular weight of 1,000-15,000, most preferably 1,500-8,000.

The viscosity reducer and the glycolide product may not be co-distilled. The pyrolysis reaction may comprise distilling the glycolide product without the viscosity reducer.

The pyrolysis reaction has a high pyrolysis conversion ratio. The term "pyrolysis conversion ratio" used herein refers to the percentage of glycolide product after a pyrolysis reaction over the total methyl polyglycolate or a methyl polyglycolate product available at the beginning of the pyrolysis reaction. The pyrolysis conversion rate of the process of the present invention is greater than 80%, 85%, 90% 95% or 99% of the glycolic acid oligomer.

The glycolide product prepared by the process of the present invention may have a free acid content of less than 10, 5, 2 or 1 wt% based on the weight of the glycolide product.

The glycolic acid oligomer prepared by the process of the present invention may have a weight average molecular weight of 4,000-80,000. The glycolic acid oligomer may have a free acid content of less than 10, 5, 2 or 1 wt% based on the weight of the glycolic acid oligomer.

Several test methods may be used to characterize the glycolide production process and the products generated throughout the process according to the present invention.

### 1. Weight average molecular weight

A sample is dissolved in a solution of 5 mmol/L sodium trifluoroacetate in hexafluoroisopropanol to prepare a solution of 0.05-0.3 wt% (mass fraction). The solution is then filtered with a 0.4 µm pore size polytetrafluoroethylene filter. 20 µL of the filtered solution is added to the gel permeation chromatography (GPC) injector for determination of molecular weight of the sample. Five standard molecular weights of methyl methacrylate with different molecular weights are used for molecular weight correction.

### 2. Free acid

0.5 g sample and about 20ml of dimethyl sulfoxide are added into an Erlenmeyer flask to make a sample solution. 0.01 mol/L potassium hydroxide solution is used to titrate the sample solution to detect the free acid of the sample.

### 3. Dynamic viscosity of pyrolysis residue

The viscosity of a sample at 230 °C was measured using a rotational viscometer.

The term "about" as used herein when referring to a measurable value such as an amount, a percentage, and the like, is meant to encompass variations of ±20% or ±10%, more preferably ±5%, even more preferably ±1%, and still more preferably ±0.1% from the specified value, as such variations are appropriate.

### Example 1. Depolymerization

### A. Depolymerized products

28 depolymerized products were prepared as described below.

Depolymerization 1: 100 g of recovered methyl polyglycolate having a weight average molecular weight of 30,000 and 100 g of methanol were placed in an autoclave, heated to 180 °C, reacted for 24 h, and cooled to normal temperature. A methyl glycolic acid oligomer was obtained from the filter cake after filtration, and had a weight average molecular weight of about 500. Methyl glycolate was detected in the filtrate. Thus, depolymerized product a was obtained.

Depolymerization 2: 100 g of recovered methyl polyglycolate having a weight average molecular weight of 30,000 and 100 g of methanol were placed in an autoclave, heated to 180 °C, reacted for 12 hours, and cooled to normal temperature. A methyl glycolic acid oligomer was obtained from the filter cake after filtration, and had a weight-average molecular weight of about 1,400. Methyl glycolate was detected in the filtrate. Thus, depolymerized product b was obtained.

Depolymerization 3: 100 g of recovered methyl polyglycolate having a weight average molecular weight of 30,000 and 100 g of ethanol were placed in an autoclave, heated to 180 °C, reacted for 24 hours, and cooled to normal temperature. A methyl glycolic acid oligomer was obtained from the filter cake after filtration, and had a weight-average molecular weight of about 300. Methyl glycolate was detected in the filtrate. Thus, depolymerized product c was obtained.

Depolymerization 4: 100 g of recovered methyl polyglycolate having a weight average molecular weight of 30,000 and 100 g of ethanol were placed in an autoclave, heated to 180 °C, reacted for 24 hours, and cooled to normal temperature. A methyl glycolic acid oligomer was obtained from the filter cake after filtration, and had a weight average molecular weight of about 450. Methyl glycolate was detected in the filtrate. Thus, depolymerized product d was obtained.

Depolymerization 5: 100 g of recovered methyl polyglycolate having a weight average molecular weight of 30,000 and 100 g of methyl glycolate were placed in an autoclave, heated to 180 °C, reacted for 24 hours, and cooled to normal temperature. A methyl glycolic acid oligomer was obtained from the filter cake after filtration, and had a weight average molecular weight of about 500. Methyl glycolate was detected in the filtrate. Thus, depolymerized product e was obtained.

Depolymerization 6: 50 g of recovered methyl polyglycolate having a weight average molecular weight of 30,000 and 50 g of recovered methyl polyglycolate having a weight average molecular weight of 80,000 were placed in an autoclave, heated to 180 °C, reacted for 24 hours, and cooled to normal temperature. A methyl glycolic acid oligomer was obtained from the filter cake after filtration, and had a weight average molecular weight of about 700. Methyl glycolate was detected in the filtrate. Thus, depolymerized product f was obtained.

Depolymerization 7: 40 g of recovered methyl polyglycolate having a weight average molecular weight of 3,000, 30 g of recovered methyl polyglycolate having a weight average molecular weight of 30,000, 30 g of recovered methyl polyglycolate having a weight average molecular weight of 80,000 and 100 g of methanol were placed in an autoclave, heated to 180 °C, reacted for 24 hours, and cooled to normal temperature. A methyl glycolic acid oligomer was obtained from the filter cake after filtration, and had a weight average molecular weight of about 500. Methyl glycolate was detected in the filtrate. Thus, depolymerized product g was obtained.

Depolymerization 8: 40 g of recovered methyl polyglycolate having a weight average molecular weight of 1,700, 30 g of recovered methyl polyglycolate having a weight average molecular weight of 80,000, 50 g of recovered methyl polyglycolate having a weight average molecular weight of 250,000 and 100 g of methanol were placed in an autoclave, heated to 180 °C, reacted for 24 hours, and cooled to normal temperature. A methyl glycolic acid oligomer was obtained from the filter cake after filtration, and had a weight average molecular weight of about 1,100. Methyl glycolate was detected in the filtrate. Thus, depolymerized product h was obtained.

Depolymerization 9: 100 g of recovered methyl polyglycolate having a weight average molecular weight of 30,000 and 100 g of methanol were placed in an autoclave, heated to 120 °C, reacted for 24 hours, and cooled to normal temperature. A methyl glycolic acid oligomer was obtained from the filter cake after filtration, and had a weight average molecular weight of about 780. Methyl glycolate was detected in the filtrate. Thus, depolymerized product i was obtained.

Depolymerization 10: 100 g of recovered methyl polyglycolate having a weight average molecular weight of 30,000 and 100 g of methanol were placed in an autoclave, heated to 120 °C, reacted for 12 hours, and cooled to normal temperature. A methyl glycolic acid oligomer was obtained from the filter cake after filtration, and had a weight average molecular weight of about 1200. Methyl glycolate was detected in the filtrate. Thus, depolymerized product j was obtained.

Depolymerization 11: 100 g of recovered methyl polyglycolate having a weight average molecular weight of 30,000 and 100 g of methanol were placed in an autoclave, heated to 120 °C, reacted with simultaneously microwave irradiation for 12 hours, and cooled to normal temperature. A methyl glycolic acid oligomer was obtained from the filter cake after filtration, and had a weight average molecular weight of about 300. Methyl glycolate was detected in the filtrate. Thus, depolymerized product k was obtained.

Depolymerization 12: 100 g of recovered methyl polyglycolate having a weight average molecular weight of 30,000 and 100 g of methanol were placed in an autoclave, heated to 120 °C, reacted with simultaneously microwave irradiation for 8 hours, and cooled to normal temperature. A methyl glycolic acid oligomer was obtained from the filter cake after filtration, and had a weight average molecular weight of about 450. Methyl glycolate was detected in the filtrate. Thus, depolymerized product I was obtained.

Depolymerization 13: 100 g of recovered methyl polyglycolate having a weight average molecular weight of 30,000, 100 g of methanol, and 0.01 g of La₂O₃ were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 190. Methyl glycolate was detected in the filtrate. Thus, depolymerized product m was obtained.

Depolymerization 14: 100 g of recovered methyl polyglycolate having a weight average molecular weight of 30,000, 100 g of methanol, and 0.01 g of Ce(HCO₃)₄ were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 160. Methyl glycolate was detected in the filtrate. Thus, depolymerized product n was obtained.

Depolymerization 15: 100 g of recovered methyl polyglycolate having a weight average molecular weight of 30,000, 100 g of methanol, and 0.01 g of lanthanum were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 210. Methyl glycolate was detected in the filtrate. Thus, depolymerized product o was obtained.

Depolymerization 16: 100 g of recovered methyl polyglycolate having a weight average molecular weight of 500,000 and 100 g of methanol were placed in an autoclave, heated to 120 °C, reacted for 12 hours, and cooled to normal temperature. A methyl glycolic acid oligomer was obtained from the filter cake after filtration, and had a weight average molecular weight of about 16,000. Methyl glycolate was detected in the filtrate. Thus, depolymerized product p was obtained.

Depolymerization 17: 100 g of recovered methyl polyglycolate having a weight average molecular weight of 500,000 and 100 g of methanol were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 6,000. Methyl glycolate was detected in the filtrate. Thus, depolymerized product q was obtained.

Depolymerization 18: 100 g of recovered methyl polyglycolate having a weight average molecular weight of 500,000 were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 1,200. Methyl glycolate was detected in the filtrate. Thus, depolymerized product r was obtained.

Depolymerization 19: 100 g of recovered methyl polyglycolate having a weight average molecular weight of 700,000 and 100 g of methanol were placed in an autoclave, heated to 120 °C, reacted for 12 hours, and cooled to normal temperature. A methyl glycolic acid oligomer was obtained from the filter cake after filtration, and had a weight average molecular weight of about 32,000. Methyl glycolate was detected in the filtrate. Thus, depolymerized product s was obtained.

Depolymerization 20: 100 g of recovered methyl polyglycolate having a weight average molecular weight of 700,000 and 100 g of methanol were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 11,000. Methyl glycolate was detected in the filtrate. Thus, depolymerized product t was obtained.

Depolymerization 21: 100 g of recovered methyl polyglycolate having a weight average molecular weight of 700,000, 100 g of methanol, and 0.01 g of Ce(HCO₃)₄ were placed in an autoclave, heated to 120 °C, reacted with simultaneously microwave irradiation for 12 hours, and cooled to normal temperature. A methyl glycolic acid oligomer was obtained from the filter cake after filtration, and had a weight average molecular weight of about 1,800. Methyl glycolate was detected in the filtrate. Thus, depolymerized product u was obtained.

Depolymerization 22: 100 g of recovered methyl polyglycolate having a weight average molecular weight of 900,000 and 100 g of methanol were placed in an autoclave, heated to 120 °C, reacted for 12 hours, and cooled to normal temperature. A methyl glycolic acid oligomer was obtained from the filter cake after filtration, and had a weight average molecular weight of about 43,000. Methyl glycolate was detected in the filtrate. Thus, depolymerized product v was obtained.

Depolymerization 23: 100 g of recovered methyl polyglycolate having a weight average molecular weight of 900,000 and 100 g of methanol were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 17,000. Methyl glycolate was detected in the filtrate. Thus, depolymerized product w was obtained.

Depolymerization 24: 100 g of recovered methyl polyglycolate having a weight average molecular weight of 900,000, 100 g of methanol, and 0.01 g of Ce(HCO₃)₄ were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 1,900. Methyl glycolate was detected in the filtrate. Thus, depolymerized product x was obtained.

Depolymerization 25: 100 g of recovered methyl polyglycolate having a weight average molecular weight of 30,000, 100 g of methanol, and 0.01 g of Ce(HCO₃)₄ were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 190. Methyl glycolate was detected in the filtrate. Thus, depolymerized product y was obtained.

Depolymerization 26: 100 g of recovered methyl polyglycolate having a weight average molecular weight of 30,000, 100 g of methanol, and 0.0002 g of Ce(HCO₃)₄ were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 300. Methyl glycolate was detected in the filtrate. Thus, depolymerized product z was obtained.

Depolymerization 27: 100 g of recovered methyl polyglycolate having a weight average molecular weight of 30,000, 100 g of methanol, and 4 g of Ce(HCO₃)₄ were placed in an autoclave, heated to 120 °C, reacted with simultaneously applied microwave irradiation for 12 hours, and cooled to normal temperature. A methyl glycolic acid oligomer was obtained from the filter cake after filtration, and had a weight average molecular weight of about 180. Methyl glycolate was detected in the filtrate. Thus, depolymerized product δ was obtained.

Depolymerization 28: 100 g of recovered methyl polyglycolate having a weight average molecular weight of 30,000, 100 g of methanol, and 4 g of Ce(HCO₃)₄ were placed in an autoclave, heated to 120 °C, reacted for 12 hours, and cooled to normal temperature. A methyl glycolic acid oligomer was obtained from the filter cake after filtration, and had a weight average molecular weight of about 820. Methyl glycolate was detected in the filtrate. Thus, depolymerized product β was obtained.

### B. Repolymerized products

Nine repolymerized products were prepared as described below.

Repolymerization 1: 100 g of the depolymerized product a was gradually heated from 160 °C to 230 °C in an autoclave. The pressure was controlled at an absolute pressure of 3 kPa. The methanol formed during the reaction was continuously removed for 2 hours. The resulting glycolic acid oligomer A had a weight average molecular weight of 6,200 and a free acid content of 1.3 wt%.

Repolymerization 2: 100 g of the depolymerized product a was gradually heated from 160 °C to 230 °C in an autoclave. The pressure was controlled at an absolute pressure of 3 kPa. The methanol formed during the reaction was continuously removed for 6 hours. The resulting glycolic acid oligomer B had a weight average molecular weight of 8,200 and a free acid content of 0.9 wt%.

Repolymerization 3: 100 g of the depolymerized product a was gradually heated from 160 °C to 230 °C in an autoclave. The pressure was controlled at an absolute pressure of 3 kPa. The methanol formed during the reaction was continuously removed for 8 hours. The resulting glycolic acid oligomer C had a weight average molecular weight of 15,000 and a free acid content of 0.7 wt%.

Repolymerization 4: 100 g of the depolymerized product a and 0.5 g of stannous octoate were gradually heated from 160 °C to 230 °C in an autoclave. The pressure was controlled at an absolute pressure of 3 kPa. The methanol formed during the reaction was continuously removed for 6 hours. The resulting glycolic acid oligomer D had a weight average molecular weight of 9,400 and a free acid content of 0.6 wt%.

Repolymerization 5: 100 g of the depolymerized product a was gradually heated from 160 °C to 230 °C in an autoclave. The pressure was controlled at an absolute pressure of 50 kPa. The methanol formed during the reaction was continuously removed for 8 hours. The resulting glycolic acid oligomer E had a weight average molecular weight of 5,500 and a free acid content of 0.7 wt%.

Repolymerization 6: 100 g of the depolymerized product d was gradually heated from 160 °C to 230 °C in an autoclave. The pressure was controlled at an absolute pressure of 3 kPa. The methanol formed during the reaction was continuously removed for 6 hours. The resulting glycolic acid oligomer F had a weight average molecular weight of 7,900 and a free acid content of 0.9 wt%.

Repolymerization 7: 100 g of the depolymerized product e was gradually heated from 160 °C to 230 °C in an autoclave. The pressure was controlled at an absolute pressure of 3 kPa. The methanol formed during the reaction was continuously removed for 6 hours. The resulting glycolic acid oligomer G had a weight average molecular weight of 7,900 and a free acid content of 0.8 wt%.

Repolymerization 8: 100 g of the depolymerized product e was gradually heated from 160 °C to 230 °C in an autoclave. The pressure was controlled at an absolute pressure of 70 kPa. The methanol formed during the reaction was continuously removed for 6 hours. The resulting glycolic acid oligomer H had a weight average molecular weight of 43,000 and a free acid content of 1.1 wt%.

Repolymerization 9: 100 g of the depolymerized product e was gradually heated from 160 °C to 230 °C in an autoclave. The pressure was controlled at an absolute pressure of 70 kPa. The methanol formed during the reaction was continuously removed for 8 hours. The resulting glycolic acid oligomer I had a weight average molecular weight of 72,000 and a free acid content of 1.3 wt%.

### C. Pyrolysis products

Five pyrolysis products were prepared as described below.

Pyrolysis 1: 100 g of the glycolic acid oligomer A was subjected to a pyrolysis reaction at 250 °C under an absolute pressure of 1 kPa for 2 hours, and distilled to a glycolide product. The glycolide product was collected by condensing it with ice water. The glycolide pyrolysis yield was 90%. The free acid content was 1.9 wt%. The dynamic viscosity of the pyrolysis residue at 230 °C was 0.35 Pa.s.

Pyrolysis 2: 100 g of the glycolic acid oligomer B was subjected to a pyrolysis reaction at 250 °C under an absolute pressure of 1 kPa for 2 hours, and distilled to a glycolide product. The glycolide product was collected by condensing it with ice water. The glycolide pyrolysis yield was 91%. The free acid content was 1.5%. The dynamic viscosity of the pyrolysis residue at 230 °C was 0.67 Pa.s.

Pyrolysis 3: 100 g of the glycolic acid oligomer C was subjected to a pyrolysis reaction at 250 °C under an absolute pressure of 1 kPa for 2 hours, and distilled to a glycolide product. The glycolide product was collected by condensing it with ice water. The glycolide pyrolysis yield was 91%. The free acid content was 1.1 wt%. The dynamic viscosity of the pyrolysis residue at 230 °C was 0.72 Pa.s.

Pyrolysis 4: 100 g of the glycolic acid oligomer C and 40 g of polyethylene glycol (molecular weight 4,000) were subjected to a pyrolysis reaction at 250 °C under an absolute pressure of 1 kPa for 2 hours, and distilled to a glycolide product. The glycolide product was collected by condensing it with ice water. The glycolide pyrolysis yield was 94%. The free acid content was 0.9 wt%. The dynamic viscosity of the pyrolysis residue at 230 °C was 0.13 Pa.s.

Pyrolysis 5: 100 g of the glycolic acid oligomer C and 40 g of paraffin wax (molecular weight 1,500) were subjected to a pyrolysis reaction at 250 °C under an absolute pressure of 1 kPa for 2 hours, and distilled to a glycolide product. The glycolide product was collected by condensing it with ice water. The glycolide pyrolysis yield was 95%. The free acid content was 1.0 wt%. The dynamic viscosity of the pyrolysis residue at 230 °C was 0.22 Pa.s.

### D. Comparative products

Two comparative products were prepared as described below.

Comparative 1: 100 g of recovered methyl polyglycolate having a weight average molecular weight of 10,000 and a free acid content of 11.4 wt% was subjected to a pyrolysis reaction at 250 °C under an absolute pressure of 1 kPa for 2 hours, and distilled to a glycolide product. The glycolide product was collected by condensing it with ice water. The glycolide pyrolysis yield was 69%. The free acid content was 15.2 wt%. The dynamic viscosity of the pyrolysis residue at 230 °C was 1.03 Pa.s.

Comparative 2: 100 g of recovered methyl polyglycolate having a weight average molecular weight of 180,000 and a free acid content of 0.9 wt% was subjected to a pyrolysis reaction at 250 °C under an absolute pressure of 1 kPa for 2 hours, and distilled to a glycolide product. The glycolide product was collected by condensing it with ice water. The glycolide pyrolysis yield was 63%. The free acid content was 2.2 wt%. The dynamic viscosity of the pyrolysis residue at 230 °C was 13.54 Pa.s.

Although the invention is illustrated and described herein with reference to specific embodiments, the invention is not intended to be limited to the details shown. Rather, various modifications may be made in the details within the scope and range of equivalents of the appended claims without departing from the invention.

## Claims

1. A process of preparing a glycolide product from a methyl polyglycolate or a methyl polyglycolate product, comprising:
(a) depolymerizing a methyl polyglycolate or methyl polyglycolate product in the presence of a depolymerization agent, whereby a depolymerized product is generated, wherein the depolymerized product comprises glycolate, glycolate polymer or glycolate prepolymer;
(b) repolymerizing the depolymerized product, whereby a repolymerized mixture is generated, wherein the repolymerized mixture comprises a glycolic acid oligomer; and
(c) pyrolyzing the repolymerized mixture, whereby a glycolide product is prepared.

2. The process of claim 1, wherein the depolymerization agent comprises one or more hydroxyl groups and comprises an alkyl alcohol, an alkyl alkyd or an alkyl alkanoate, wherein:
the alkyl alcohol preferably comprises one hydroxyl group;
optionally, the alkyl alkyd comprises one or more hydroxyl groups and one or more carboxyl groups at a number ratio of the hydroxyl groups to the carboxyl groups in the range from 0.1:1 to 10:1;
optionally, the alkyl alkanoate comprises one or more hydroxyl groups and one or more ester groups at a number ratio of the hydroxyl groups to the ester groups in the range from 0.1:1 to 10:1.

3. The process of claim 1, wherein step (a) is carried out at 25-260 °C for 0.5-48 hours.

4. The process of claim 1, further comprising applying microwave irradiation to control temperature in step (a), whereby depolymerization is accelerated.

5. The process of claim 1, wherein step (a) is carried out in the presence of a rare earth metal catalyst, wherein the rare earth metal catalyst is a metal oxide, a rare earth metal inorganic salt or a rare earth metal complex comprising lanthanum (La), cerium (Ce), praseodymium (Pr), neodynium (Nd), promethium (Pm), strontium (Sr), samarium (Sm), europium (Eu), gadolinium (Gd), terbium (Tb), dysprosium (Dy), holmium (Ho), erbium (Er), thulium (Tm), ytterbium (Yb), lutetium (Lu), yttrium (Y), scandium (Sc), or a combination thereof,
wherein the rare earth metal catalyst is optionally present in an amount of 0.0001-5 wt%, based on the weight of the methyl polyglycolate or the methyl polyglycolate product.

6. The process of claim 1, wherein the glycolic acid oligomer has a weight average molecular weight of 4,000-80,000 and a free acid content of less than 2 wt%,
wherein the weight average molecular weight is determined by gel permeation chromatography (GPC) as described in the specification; and
wherein the free acid content is measured by the method described in the specification.

7. The process of claim 1, wherein step (b) is carried out in the presence of a repolymerization catalyst selected from the group consisting of zinc-based compounds, tin-based compounds, lanthanide-based compounds, and titanium-based compounds.

8. The process of claim 1, wherein step (b) is carried out at 160-250 °C and under an absolute pressure of 0.05-50.00 kPa.

9. The process of claim 1, wherein step (c) is carried out at 230-280 °C and an absolute pressure of 0.05-30.00 kPa.

10. The process of claim 1, wherein step (c) is carried out in the presence of a viscosity reducer, and further comprising distilling the glycolide product without the viscosity reducer, wherein:
optionally, the viscosity reducer is present in an amount of 5-500 wt% based on the weight of the methyl polyglycolate or the methyl polyglycolate product;
optionally, the viscosity reducer is a polyether polyol or a hydrocarbon mixture,
wherein the polyether polyol is a polyethylene glycol, polypropylene glycol or polybutylene glycol, and
the hydrocarbon mixture has a weight average molecular weight of 1,000-15,000, wherein the weight average molecular weight is determined by gel permeation chromatography (GPC) as described in the specification.

11. The process of claim 1, wherein the methyl polyglycolate or the methyl polyglycolate product has a weight average molecular weight of 1,000-1,000,000, wherein the weight average molecular weight is determined by gel permeation chromatography (GPC) as described in the specification.

12. The process of claim 1, wherein step (c) has a pyrolysis conversion rate of more than 90% of the glycolic acid oligomer and generates an incompletely converted glycolic acid oligomer; and
optionally, further comprises discharging the incompletely converted glycolic acid oligomer as a residue at 230 °C with a dynamic viscosity less than 1 Pa·s, wherein the dynamic viscosity is measured using a rotational viscometer at 230°C.

13. A process for preparing a glycolic acid oligomer from a methyl polyglycolate or a methyl polyglycolate product, wherein the process comprises:
(a) depolymerizing a methyl polyglycolate or methyl polyglycolate product in the presence of a depolymerization agent, whereby a depolymerized product is generated, wherein the depolymerized product comprises glycolate, glycolate polymer or glycolate prepolymer; and
(b) repolymerizing the depolymerized product, whereby a glycolic acid oligomer is prepared.

14. The process of claim 13, wherein the glycolic acid oligomer has a weight average molecular weight of 4,000-80,000, wherein the weight average molecular weight is determined by gel permeation chromatography (GPC) as described in the specification.

15. The process of claim 13, wherein the glycolic acid oligomer has a free acid content of less than 2 wt%, wherein the free acid content is measured by the method described in the specification.

## Patentansprüche

1. Verfahren zum Herstellen eines Glykolidprodukts aus einem Methylpolyglykolat oder einem Methylpolyglykatprodukt, umfassend:
(a) Depolymerisieren eines Methylpolyglykolats oder Methylpolyglykatprodukts in Gegenwart eines Depolymerisationsmittels, wodurch ein depolymerisiertes Produkt erzeugt wird, wobei das depolymerisierte Produkt Glykolat, Glykolatpolymer oder Glykolatpräpolymer umfasst;
(b) Repolymerisieren des depolymerisierten Produkts, wodurch ein repolymerisiertes Gemisch erzeugt wird, wobei das repolymerisierte Gemisch ein Glykolsäureoligomer umfasst; und
(c) Pyrolysieren des repolymerisierten Gemischs, wodurch ein Glykolidprodukt hergestellt wird.

2. Verfahren nach Anspruch 1, wobei das Depolymerisationsmittel eine oder mehrere Hydroxylgruppen umfasst und einen Alkylalkohol, ein Alkylalkid oder ein Alkylalkanoat umfasst, wobei:
der Alkylalkohol vorzugsweise eine Hydroxylgruppe umfasst;
optional das Alkylalkid eine oder mehrere Hydroxylgruppen und eine oder mehrere Carboxylgruppen in einem Zahlenverhältnis der Hydroxylgruppen zu den Carboxylgruppen im Bereich von 0,1:1 bis 10:1 umfasst;
optional das Alkylalkanoat eine oder mehrere Hydroxylgruppen und eine oder mehrere Estergruppen in einem Zahlenverhältnis der Hydroxylgruppen zu den Estergruppen im Bereich von 0,1:1 bis 10:1 umfasst.

3. Verfahren nach Anspruch 1, wobei Schritt (a) bei 25-260 °C für 0,5-48 Stunden ausgeführt wird.

4. Verfahren nach Anspruch 1, ferner umfassend Anwenden von Mikrowellenbestrahlung, um die Temperatur in Schritt (a) zu steuern, wodurch die Depolymerisation beschleunigt wird.

5. Verfahren nach Anspruch 1, wobei Schritt (a) in Gegenwart eines Seltenerdmetallkatalysators ausgeführt wird, wobei der Seltenerdmetallkatalysator ein Metalloxid, ein anorganisches Seltenerdmetallsalz oder ein Seltenerdmetallkomplex ist, umfassend Lanthan (La), Cer (Ce), Praseodym (Pr), Neodym (Nd), Promethium, (Pm), Strontium (Sr), Samarium (Sm), Europium (Eu), Gadolinium (Gd), Terbium (Tb), Dysprosium (Dy), Holmium (Ho), Erbium (Er), Thulium (Tm), Ytterbium (Yb), Lutetium (Lu), Yttrium (Y), Scandium (Sc) oder eine Kombination davon,
wobei der Seltenerdmetallkatalysator optional in einer Menge von 0,0001-5 Gew.-% vorliegt, basierend auf dem Gewicht des Methylpolyglykolats oder des Methylpolyglykolatprodukts.

6. Verfahren nach Anspruch 1, wobei das Glykolsäureoligomer ein gewichtsmittleres Molekulargewicht von 4.000-80.000 und einen freien Säuregehalt von weniger als 2 Gew.-% hat,
wobei das gewichtsmittlere Molekulargewicht durch Gelpermeationschromatographie (GPC), wie in der Beschreibung beschrieben, bestimmt wird; und
wobei der freie Säuregehalt durch das in der Beschreibung beschriebene Verfahren gemessen wird.

7. Verfahren nach Anspruch 1, wobei Schritt (b) in Gegenwart eines Repolymerisationskatalysators ausgeführt wird, ausgewählt aus der Gruppe bestehend aus zinkbasierten Verbindungen, zinnbasierten Verbindungen, lanthanidbasierten Verbindungen und titanbasierten Verbindungen.

8. Verfahren nach Anspruch 1, wobei Schritt (b) bei 160-250 °C und unter einem absoluten Druck von 0,05-50,00 kPa ausgeführt wird.

9. Verfahren nach Anspruch 1, wobei Schritt (c) bei 230-280 °C und einem absoluten Druck von 0,05-30,00 kPa ausgeführt wird.

10. Verfahren nach Anspruch 1, wobei Schritt (c) in Gegenwart eines Viskositätsreduzierers ausgeführt wird, und ferner umfassend ein Destillieren des Glykolidprodukts ohne den Viskositätsreduzierer, wobei:
optional der Viskositätsreduzierer in einer Menge von 5-500 Gew.-% vorliegt, basierend auf dem Gewicht des Methylpolyglykolats oder des Methylpolyglykolatprodukts;
optional der Viskositätsreduzierer ein Polyetherpolyol oder ein Kohlenwasserstoffgemisch ist,
wobei das Polyetherpolyol ein Polyethylenglykol, Polypropylenglykol oder Polybutylenglykol ist, und
das Kohlenwasserstoffgemisch ein gewichtsmittleres Molekulargewicht von 1.000-15.000 hat, wobei das gewichtsmittlere Molekulargewicht durch Gelpermeationschromatographie (GPC), wie in der Beschreibung beschrieben, bestimmt wird.

11. Verfahren nach Anspruch 1, wobei das Methylpolyglykolat oder das Methylpolyglykolatprodukt ein gewichtsmittleres Molekulargewicht von 1.000-1.000.000 hat, wobei das gewichtsmittlere Molekulargewicht durch Gelpermeationschromatographie (GPC), wie in der Beschreibung beschrieben, bestimmt wird.

12. Verfahren nach Anspruch 1, wobei Schritt (c) eine Pyrolyseumwandlungsrate von mehr als 90 % des Glykolsäureoligomers hat und ein unvollständig umgewandeltes Glykolsäureoligomer erzeugt; und
optional ferner umfassend ein Abführen des unvollständig umgewandelten Glykolsäureoligomers als einen Rückstand bei 230 °C mit einer dynamischen Viskosität von weniger als 1 Pa·s, wobei die dynamische Viskosität unter Verwendung eines Rotationsviskosimeters bei 230 °C gemessen wird.

13. Verfahren zum Herstellen eines Glykolsäureoligomers aus einem Methylpolyglykolat oder einem Methylpolyglykatprodukt, wobei das Verfahren umfasst:
(a) Depolymerisieren eines Methylpolyglykolats oder Methylpolyglykatprodukts in Gegenwart eines Depolymerisationsmittels, wodurch ein depolymerisiertes Produkt erzeugt wird, wobei das depolymerisierte Produkt Glykolat, Glykolatpolymer oder Glykolatpräpolymer umfasst; und
(b) Repolymerisieren des depolymerisierten Produkts, wodurch ein Glykolsäureoligomer hergestellt wird.

14. Verfahren nach Anspruch 13, wobei das Glykolsäureoligomer ein gewichtsmittleres Molekulargewicht von 4.000-80.000 hat, wobei das gewichtsmittlere Molekulargewicht durch Gelpermeationschromatographie (GPC), wie in der Beschreibung beschrieben, bestimmt wird.

15. Verfahren nach Anspruch 13, wobei das Glykolsäureoligomer einen freien Säuregehalt von weniger als 2 Gew.-% hat, wobei der freie Säuregehalt durch das in der Beschreibung beschriebene Verfahren gemessen wird.

## Revendications

1. Procédé de préparation d'un produit de glycolide à partir d'un poly(glycolate de méthyle) ou d'un produit de poly(glycolate de méthyle), comprenant :
(a) la dépolymérisation d'un poly(glycolate de méthyle) ou d'un produit de poly(glycolate de méthyle) en présence d'un agent de dépolymérisation, moyennant quoi un produit dépolymérisé est généré, lequel produit dépolymérisé comprend un glycolate, un polymère de glycolate ou un prépolymère de glycolate ;
(b) la repolymérisation du produit dépolymérisé, moyennant quoi un mélange repolymérisé est généré, lequel mélange repolymérisé comprend un oligomère d'acide glycolique ; et
(c) la pyrolyse du mélange repolymérisé, moyennant quoi un produit de glycolide est préparé.

2. Procédé selon la revendication 1, dans lequel l'agent de dépolymérisation comprend un ou plusieurs groupes hydroxyle et comprend un alcool alkylique, un acide hydroxycarboxylique alkylé ou un alcanoate d'alkyle, dans lequel
l'alcool alkylique comprend de préférence un seul groupe hydroxyle ;
éventuellement l'acide hydroxycarboxylique alkylé comprend un ou plusieurs groupes hydroxyle et un ou plusieurs groupes carboxyle en un rapport en nombre des groupes hydroxyle aux groupes carboxyle situé dans la plage allant de 0,1/1 à 10/1 ;
éventuellement l'alcanoate d'alkyle comprend un ou plusieurs groupes hydroxyle et un ou plusieurs groupes ester en un rapport en nombre des groupes hydroxyle aux groupes ester situé dans la plage allant de 0,1/1 à 10/1.

3. Procédé selon la revendication 1, dans lequel l'étape (a) est effectuée à une température de 25 à 260°C pendant 0,5 à 48 heures.

4. Procédé selon la revendication 1, comprenant en outre l'application d'une irradiation de micro-ondes pour réguler la température dans l'étape (a), moyennant quoi la dépolymérisation est accélérée.

5. Procédé selon la revendication 1, dans lequel l'étape (a) est effectuée en présence d'un catalyseur à base de métal des terres rares, lequel catalyseur à base de métal des terres rares est un oxyde métallique, un sel inorganique de métal des terres rares ou un complexe de métal des terres rares comprenant du lanthane (La), du cérium (Ce), du praséodyme (Pr), du néodyme (Nd), du prométhium (Pm), du strontium (Sr), du samarium (Sm), de l'europium (Eu), du gadolinium (Gd), du terbium (Tb), du dysprosium (Dy), du holmium (Ho), de l'erbium (Er), du thulium (Tm), de l'ytterbium (Yb), du lutécium (Lu), de l'yttrium (Y), du scandium (Sc), ou une de leurs combinaisons,
dans lequel le catalyseur à base de métal des terres rares est éventuellement présent en une quantité de 0,0001 à 5 % en poids, par rapport au poids du poly(glycolate de méthyle) ou du produit de poly(glycolate de méthyle).

6. Procédé selon la revendication 1, dans lequel l'oligomère d'acide glycolique a une masse moléculaire moyenne en masse de 4 000 à 80 000 et une teneur en acide libre inférieure à 2 % en poids,
dans lequel la masse moléculaire moyenne en masse est déterminée par chromatographie d'exclusion diffusion (GPC) comme décrit dans le fascicule ; et
dans lequel la teneur en acide libre est mesurée par la méthode décrite dans le fascicule.

7. Procédé selon la revendication 1, dans lequel l'étape (b) est effectuée en présence d'un catalyseur de repolymérisation choisi dans le groupe constitué par les composés à base de zinc, les composés à base d'étain, les composés à base de lanthanide, et les composés à base de titane.

8. Procédé selon la revendication 1, dans lequel l'étape (b) est effectuée à une température de 160 à 250°C et sous une pression absolue de 0,05 à 50,00 kPa.

9. Procédé selon la revendication 1, dans lequel l'étape (c) est effectuée à une température de 230 à 280°C et sous une pression absolue de 0,05 à 30,00 kPa.

10. Procédé selon la revendication 1, dans lequel l'étape (c) est effectuée en présence d'un réducteur de viscosité, et comprenant en outre la distillation du produit de glycolide sans le réducteur de viscosité, dans lequel :
éventuellement le réducteur de viscosité est présent en une quantité de 5 à 500 % en poids par rapport au poids du poly(glycolate de méthyle) ou du produit de poly(glycolate de méthyle) ;
éventuellement le réducteur de viscosité est un polyéther-polyol ou un mélange d'hydrocarbures,
dans lequel le polyéther-polyol est un polyéthylèneglycol, un polypropylèneglycol ou un polybutylèneglycol, et
le mélange d'hydrocarbures a une masse moléculaire moyenne en masse de 1 000 à 15 000, dans lequel la masse moléculaire moyenne en masse est déterminée par chromatographie d'exclusion diffusion (GPC) comme décrit dans le fascicule.

11. Procédé selon la revendication 1, dans lequel le poly(glycolate de méthyle) ou le produit de poly(glycolate de méthyle) a une masse moléculaire moyenne en masse de 1 000 à 1 000 000, dans lequel la masse moléculaire moyenne en masse est déterminée par chromatographie d'exclusion diffusion (GPC) comme décrit dans le fascicule.

12. Procédé selon la revendication 1, dans lequel l'étape (c) a un taux de conversion par pyrolyse supérieur à 90 % de l'oligomère d'acide glycolique et génère un oligomère d'acide glycolique incomplètement converti ; et
éventuellement comprend en outre la décharge de l'oligomère d'acide glycolique incomplètement converti sous la forme d'un résidu à 230°C avec une viscosité dynamique inférieure à 1 Pa·s, dans lequel la viscosité dynamique est mesurée au moyen d'un viscosimètre rotatif à 230°C.

13. Procédé pour préparer un oligomère d'acide glycolique à partir d'un poly(glycolate de méthyle) ou d'un produit de poly(glycolate de méthyle), lequel procédé comprend :
(a) la dépolymérisation d'un poly(glycolate de méthyle) ou d'un produit de poly(glycolate de méthyle) en présence d'un agent de dépolymérisation, moyennant quoi un produit dépolymérisé est généré, lequel produit dépolymérisé comprend un glycolate, un polymère de glycolate ou un prépolymère de glycolate ; et
(b) la repolymérisation du produit dépolymérisé, moyennant quoi un oligomère d'acide glycolique est préparé.

14. Procédé selon la revendication 13, dans lequel l'oligomère d'acide glycolique a une masse moléculaire moyenne en masse de 4 000 à 80 000, dans lequel la masse moléculaire moyenne en masse est déterminée par chromatographie d'exclusion diffusion (GPC) comme décrit dans le fascicule.

15. Procédé selon la revendication 13, dans lequel l'oligomère d'acide glycolique a une teneur en acide libre inférieure à 2 % en poids, dans lequel la teneur en acide libre est mesurée par le procédé décrit dans le fascicule.
